# EUROPÄISCHE PATENTSCHRIFT

(11) **EP 1 686 112 B1**
(45) Veröffentlichungstag und Bekanntmachung des Hinweises auf die Patenterteilung: **26.06.2019**
(21) Anmeldenummer: 06000883.6
(22) Anmeldetag: 17.01.2006
(51) Int. Cl.: C07C 263/20, C07C 265/14

(54) **VERFAHREN ZUR DESTILLATION EINES GEMISCHES ISOMERER DIISOCYANATODIPHENYLMETHANE**
PROCESS FOR DISTILLING A MIXTURE OF ISOMERIC DIISOCYANATODIPHENYLMETHANES
PROCÉDÉ DE DISTILLATION D'UN MÉLANGE DE DIISOCYANATODIPHENYLMÉTHANES ISOMÉRIQUES

(30) Priorität: 29.01.2005 DE 102005004170
(43) Veröffentlichungstag der Anmeldung: 02.08.2006
(73) Patentinhaber: Covestro Deutschland AG, 51373 Leverkusen (DE)
(72) Erfinder: SCHAL, Hans-Peter, 41539 Dormagen (DE); WOLF, Ulrich, 47647 Kerken (DE)
(74) Vertreter: Levpat

(56) Entgegenhaltungen:
- EP-A- 1 475 367
- DE-A1- 10 260 092

## Beschreibung

Die Erfindung betrifft ein Verfahren zur Destillation eines Gemisches wenigstens bestehend aus 2,2'-Diisocyanatodiphenylmethan (2,2'-MDI), 2,4'-Diisocyanatodiphenylmethan (2,4'-MDI) und 4,4'-Diisocyanatodiphenylmethan (4,4'-MDI) zur Gewinnung von an 2,2'-Diisocyanatodiphenylmethan armem 2,4'-Diisocyanatodiphenylmethan sowie von an 2,2'-Diisocyanatodiphenylmethan armen Gemischen aus 4,4'- und 2,4'-Diisocyanatodiphenylmethan.

Diisocyanatodiphenylmethan-Isomere sind Bestandteile von Polyisocyanatgemischen der Diphenylmethanreihe, welche bei der Phosgenierung von Anilin/Formaldehyd-Kondensaten, im Folgenden auch Polyaminopolyphenylpolymethane genannt, entstehen.

Die Kondensation von Anilin und Formaldehyd sowie die Phosgenierung der Polyaminopolyphenylpolymethane sind aus dem Stand der Technik hinlänglich bekannt. Nach der Phosgenierung von Polyaminopolyphenylpolymethanen wird zunächst Phosgen vollständig entfernt. Dann werden die höheren Homologen des Diisocyanatodiphenylmethans (auch Polyisocyanatopolyphenylpolymethane genannt) abgetrennt. Aus dem verbleibenden Gemisch isomerer Diisocyanatodiphenylmethane, hauptsächlich bestehend aus 2,2'-Diisocyanatodiphenylmethan, 2,4'-Diisocyanatodiphenylmethan und 4,4'-Diisocyanatodiphenylmethan (nachfolgend auch vereinfachend als MDI-Isomerengemisch oder MDI-Rohgemisch bezeichnet), können anschließend reine Diisocyanatodiphenylmethan-Isomere oder Gemische aus zwei oder drei Isomeren nach gewünschten Produktspezifikationen abgetrennt werden. Für die Abtrennung sind verschiedene Verfahren auf Basis einer Destillation oder Kristallisation oder einer Kombination aus Destillation und Kristallisation aus dem Stand der Technik bekannt.

Beispielsweise beschreibt WO 02/070581 ein Verfahren zur Herstellung von 2,4'-Diisocyanatodiphenylmethan durch Abtrennen aus einem MDI-Rohgemisch.

Ferner ist aus DE 2 631 168 A ein Verfahren zur Herstellung von Diisocyanatodiphenylmethan-Isomeren durch destillative Abtrennung aus einem durch Phosgenierung von Anilin/Formaldehyd-Kondensaten gewonnenen Polyisocyanatgemisch bekannt. Dabei werden die Wertstoffe 4,4'-Diisocyanatodiphenylmethan sowie 2,4'-Diisocyanatodiphenylmethan gewonnen. Die Abtrennung von 2,2'-Diisocyanatodiphenylmethan ist nicht beschrieben.

EP 1 475 367 A1 beschreibt ein Verfahren zur Destillation eines Gemisches isomerer Diisocyanatodiphenylmethane, wenigstens bestehend aus 2,2'-Diisocyanatodiphenylmethan, 2,4'-Diisocyanatodiphenylmethan und 4,4'-Diisocyanatodiphenylmethan, wobei die Destillation mindestens einstufig ausgeführt ist und in mindestens einer Destillationsstufe eine Trennwandkolonne eingesetzt wird. Der Anmeldung ist nicht zu entnehmen, die Konzentration an 2,4'-Diisocyanatodiphenylmethan im Seitenstrom auf einen Wert im Bereich von 95 bis 99 Gew.-% einzustellen.

Für viele Anwendungen ist die Gegenwart von 2,2'-Diisocyanatodiphenylmethan in den Wertstoffen oder Wertstoffgemischen nicht erwünscht. Bei einfachen Destillationen von rohen Diisocyanatodiphenylmethangemischen ist jedoch eine Abtrennung von 2,2'-MDI nicht oder nur unter erheblichem technischen und ökonomischen Aufwand möglich.

Aufgabe der vorliegenden Erfindung ist es daher, ein ökonomisches und technisch einfaches Verfahren zur Destillation eines Gemisches isomerer Diisocyanatodiphenylmethane, wenigstens bestehend aus 2,2'-Diisocyanatodiphenylmethan, 2,4'-Diisocyanatodiphenylmethan und 4,4'-Diisocyanatodiphenylmethan (im Folgenden Ausgangsgemisch genannt) bereitzustellen, welches die Herstellung von 2,4'-MDI erlaubt, das frei von 2,2'-MDI ist oder zumindest nur einen sehr geringen 2,2'-MDI-Gehalt in der Größenordnung von maximal 0,2 Gew.-% aufweist.

Gegenstand der Erfindung ist ein Verfahren zur Herstellung von an 2,2'-MDI armem 2,4'-MDI durch Destillation entweder eines Ausgangsgemisches isomerer Diisocyanatodiphenylmethane, wenigstens bestehend aus 2,2'-Diisocyanatodiphenylmethan, 2,4'-Diisocyanatodiphenylmethan und 4,4'-Diisocyanatodiphenylmethan, oder eines in einer destillativen Vortrennung des Ausgangsgemisches erhaltenen Gemisches, welches zu 0 bis 15 Gew.-% aus 2,2'-MDI, zu 12 bis 60 Gew.-% aus 2,4'-MDI und zu 25 bis 88 Gew.-% aus 4,4'-MDI besteht, wobei die Destillation mindestens einstufig ausgeführt ist und in mindestens einer Destillationsstufe eine Trennwandkolonne eingesetzt wird, wobei das Ausgangsgemisch oder das nach destillativer Vortrennung vorliegende Diisocyanatodiphenylmethan-Isomerengemisch als Feedstrom vollständig auf die Vorfraktionierzone der Trennwandkolonne geführt wird, dadurch gekennzeichnet, dass die Flüssigkeitsaufteilung zwischen Vorfraktionierzone und Hauptfraktionierzone der Trennwandkolonne so vorgenommen wird, dass im Seitenstrom ein Gemisch erhalten wird, welches maximal 0,2 Gew.-% 2,2'-Diisocyanatodiphenylmethan und 95 bis 99 Gew.-%, bevorzugt 95 bis 98 Gew.-%, besonders bevorzugt 97,5 bis 98 Gew.-% 2,4'-Diisocyanatodiphenylmethan enthält, wobei sich die Gewichtsprozentangaben auf die Zusammensetzung der Isomere beziehen.

Sofern nicht anders angegeben, handelt es sich hier und im Folgenden bei Prozentangaben um Gewichtsprozent, wobei sich die Prozentangaben immer auf die Zusammensetzung der Isomere beziehen.

Das bei dem erfindungsgemäßen Verfahren eingesetzte Gemisch isomerer Diisocyanatodiphenylmethane entsteht bei der Phosgenierung von Polyaminopolyphenylpolymethanen, welche durch Kondensation von Anilin und Formaldehyd hergestellt werden, zu Polyisocyanatopolyphenylpolymethanen. Nach der Phosgenierung, welche vorzugsweise in Monochlorbenzol (MCB) als Lösungsmittel durchgeführt wird, werden auf destillativem Wege zunächst das Lösungsmittel und Phosgen weitgehend entfernt.

Dann wird mittels Destillation in einer an sich bekannten, z.B. aus DE 2 631 168 A, Polymerabtrennung ein Gemisch einerseits aus Polyisocyanatopolyphenylpolymethanen und Diisocyanatodiphenylmethanen und andererseits das Ausgangsgemisch der drei Isomeren 2,2'-Diisocyanatodiphenylmethan, 2,4'-Diisocyanatodiphenylmethan und 4,4'-Diisocyanatodiphenylmethan erhalten. Nach diesem Verfahrensschritt kann das Ausgangsgemisch zusätzlich enthalten: Lösungsmittel, z.B. Chlorbenzol, und andere leichter siedende Verbindungen, z.B. Phenylisocyanat, mit einem Anteil von weniger als 2 Gew.-%, maximal 5 Gew.-% Polyisocyanatopolyphenylmethane sowie maximal 5 Gew.-% höher molekulare Verbindungen, die aufgrund thermischer Belastung entstanden sind.

Das erfindungsgemäße Verfahren beruht auf der destillativen Abtrennung der MDI-Isomeren aus dem Ausgangsgemisch in einem ein- oder mehrstufigen Verfahren mittels einer Trennwandkolonne in wenigstens einer der Destillationsstufen. Mit Hilfe des erfindungsgemäßen Verfahrens gelingt die Auftrennung eines MDI-Rohgemisches in reines 2,4'-Diisocyanatodiphenylmethan und ein Gemisch aus 2,4'-Diisocyanatodiphenylmethan und 4,4'-Diisocyanatodiphenylmethan, wobei die Trennwandkolonne für unterschiedliche Feed-Zusammensetzungen und Produktspezifikationen ausgelegt werden kann. Andererseits ist für viele Anwendungszwecke der Gehalt an 4,4'-MDI von geringerer Bedeutung, so dass die Abtrennung von 2,4'-MDI nicht die vollständige Trennung von 4,4'-MDI bedeuten muss. Der Vorteil des erfindungsgemäßen Verfahrens liegt in der Verwendung einer Trennwandkolonne, womit niedrigere Herstellkosten aufgrund des geringeren technischen Aufwands gegenüber bekannten Destillationsverfahren ohne Trennwandkolonne verbunden sind.

Der Einsatz mindestens einer Trennwandkolonne zur Gewinnung von 2,4'-MDI aus einem Gemisch isomerer Diisocyanatodiphenylmethane ermöglicht es, im Vergleich zu Destillationsverfahren nach dem Stand der Technik einen bzw. zwei Destillationsschritte einzusparen. Damit ist einerseits der apparative Aufwand erheblich geringer, da neben der Destillationskolonne auch zusätzliche Wärmetauscher, Verrohrungen, usw. eingespart werden. Andererseits reduziert sich dadurch der Energieaufwand in bedeutendem Maße, da weniger Wärme zugeführt werden muss. Des Weiteren ist bei einer geringeren Anzahl von Destillationsschritten aufgrund der geringeren thermischen Belastung der durch Reaktionen der Isocyanatgruppen bedingte Rückstandsanteil geringer.

Die Destillation eines Mehrkomponentengemisches in einer Trennwandkolonne ist beispielsweise aus US 2 471 134 bekannt. In einer Trennwandkolonne verläuft eine Trennwand vertikal im mittleren Teil der Kolonne. Dadurch wird die Kolonne in vier Zonen unterteilt: eine Vorfraktionierungszone und eine Hauptfraktionierungszone im Bereich der Trennwand sowie eine Sumpfzone (Abtriebszone) und eine Rektifizierzone (Kopfzone). In die Vorfraktionierungszone wird der Mehrkomponentenstrom eingespeist. Nachfolgend wird der Teil der Vorfraktionierungszone oberhalb der Einspeisung auch als Zone A, der Teil unterhalb der Einspeisung als Zone B bezeichnet. Das Kopfprodukt wird der Rektifizierzone (Zone C), das Sumpfprodukt der Abtriebszone (Zone F) entnommen. Der Hauptfraktionierungszone wird ein intermediäres Produkt entnommen, wobei der Teil der Hauptfraktionierungszone oberhalb der Ausspeisung auch als Zone D, der Teil unterhalb der Ausspeisung als Zone E bezeichnet wird.

Üblicherweise wird bei einer Monomer-Polymertrennung ein 2-Kern-Gemisch erhalten, das zu 0 bis 5 Gew.-% aus 2,2'-MDI, zu 5 bis 15 Gew.-% aus 2,4'-MDI und zu 80 bis 95 Gew.-% aus 4,4'-MDI besteht. Ein solches Gemisch (auch Ausgangsgemisch genannt) kann bei der erfindungsgemäßen Destillation eingesetzt werden. In einer Ausführungsform des erfindungsgemäßen Verfahrens findet jedoch ein zusätzlicher Destillationsschritt statt, in dem das MDI-Isomerengemisch aus der Monomer-Polymertrennung (Ausgangsgemisch) einer Isomerendestillation (destillative Vortrennung) zugeführt wird. Dieser zusätzliche Destillationsschritt, der dem Destillationsverfahren gemäß Anspruch 1 vorgeschaltet ist, führt zu einem Gemisch, welches zu 0 bis 15 Gew.-% aus 2,2'-MDI, zu 12 bis 60 Gew.-% aus 2,4'-MDI und zu 25 bis 88 Gew.-% aus 4,4'-MDI besteht. Es kann als Feedstrom zur Trennwandkolonne eingesetzt werden. Bevorzugt besteht dieses Gemisch, das als Feedstrom zur Trennwandkolonne dienen kann, aus maximal 3,0 % 2,2'-MDI, 30 bis 60% (z.B. 49,9%) 2,4'-MDI und 40 bis 70% 4,4'-MDI (z.B. 46,9%) sowie Spuren von Leichtsiedern.

Das Ausgangsgemisch oder das MDI-Isomerengemisch nach destillativer Vortrennung wird nach dem erfindungsgemäßen Verfahren der Trennwandkolonne seitlich im Bereich der Trennwand zugeführt. Die Trennwand befindet sich im mittleren Bereich der Kolonne und teilt den Querschnitt der Kolonne. Die Länge der Trennwand ist abhängig von den Prozessbedingungen und von den Eigenschaften der eingesetzten Stoffaustauschelemente.

Die Anzahl der erforderlichen Trennstufen in den einzelnen Kolonnenabschnitten ist abhängig von den Prozessbedingungen, insbesondere der Zusammensetzung des Zulaufes und der gewünschten Produkte. So ist z.B. bei großzügig ausgelegten Kopf- und Sumpfspezifikationen eine Stoffaustauschzone im Kopfteil und Sumpfteil verzichtbar und im Bereich der Trennwand minimierbar.

In einer besonders bevorzugten Ausführungsform ergibt sich bei dem Einsatz einer Gewebepackung mit z.B. 500 m²/m³ spezifischer Oberfläche eine Trennwandlänge von ca. 6 bis 8 m. Die Stoffaustauschzonen im Kopf und Sumpf der Kolonne sind jeweils 1,20 bis 2,50 m, z.B. 1,80 m, hoch und haben jeweils 6 bis 12, z.B. 8, Trennstufen. Die Vorfraktionierzone hat oberhalb der Einspeisung 10 bis 14 Trennstufen und unterhalb der Einspeisung 13 bis 17 Trennstufen. Die Hauptfraktionierzone hat oberhalb der Ausspeisung des Seitenstroms 14 bis 18 Trennstufen und unterhalb der Ausspeisung 12 bis 16 Trennstufen.

Das Ausgangsgemisch oder das MDI-Isomerengemisch nach destillativer Vortrennung wird als Feedstrom vollständig auf die Vorfraktionierzone der Trennwandkolonne geführt. Der Sumpfstrom der Trennwandkolonne wird einem Verdampfer zugeführt und teilweise verdampft. Der Dampfstrom aus dem Verdampfer wird in die Trennwandkolonne zurückgeführt, durchströmt zunächst die Packung der Sumpfzone und teilt sich entsprechend den Druckverhältnissen auf Vorfraktionier- und Hauptfraktionierzone auf. Die Dampfströme aus diesen Zonen vereinigen sich beim Verlassen der Trennwandzone und durchströmen zuletzt die Kopfzone. Die Brüden am Kopf der Trennwandkolonne werden kondensiert und zu 88 bis 99,6 %, bevorzugt 98 bis 99%, z.B. 98, 5%, in die Kopfzone zurückgegeben. Die zurücklaufende Flüssigkeit wird unterhalb der Kopfzone zu 20 bis 60%, bevorzugt 30 bis 50%, z.B. 40%, in die Vorfraktionierzone und zu 40 bis 80%, bevorzugt 50 bis 70 %, z.B. 60%, in die Hauptfraktionierzone geleitet. Der Seitenstrom wird zunächst vollständig abgezogen und dann zu 60 bis 97 %, bevorzugt 75 bis 85%, z.B. 80%, in die Hauptfraktionierzone zurückgegeben. Die Dampfströmung in der Vorfraktionierzone und der Hauptfraktionierzone stellt sich entsprechend den Packungsdruckverlusten ein. Der Gesamtdruck an den Ein- und Austrittsbereichen der Trennwand ist für beide Zonen gleich. Falls aus prozesstechnischer Sicht im Bereich der Trennwand eine Zone stärker mit Dampf beaufschlagt werden soll, können die Querschnitte von Vorfraktionierzone und Hauptfraktionierzone auch unterschiedlich gewählt werden. Durch entsprechende Wahl der Teilquerschnitte der beiden Zonen kann der Prozess hinsichtlich des Energiebedarfes optimiert werden.

Als Stoffaustauschelemente sind Packungen besonders geeignet. Verwendbar sind aber auch andere in der Destillationstechnik bekannte Stoffaustauschelemente, wie z.B. Füllkörper oder Böden.

Die Trennwandkolonne wird hinsichtlich Druck und Temperatur unter ähnlichen Prozessbedingungen betrieben wie eine Kolonne der konventionellen Destillationstechnik. Der absolute Betriebsdruck einer solchen Kolonne wird bei 0,5 bis 30 mbar eingestellt, der Kopfdruck beträgt bevorzugt 3 bis 16 mbar. Je nach Gemischzusammensetzung beträgt die Kopftemperatur 165 bis 205 C. Der Sumpfdruck beträgt bevorzugt 9 bis 25 mbar bei Temperaturen von 205 bis 225 C.

Bei der erfindungsgemäßen Destillation des Gemisches isomerer Diisocyanatodiphenylmethane in der Trennwandkolonne wird als Sumpf ein Strom ausgeschleust, der mindestens 80% des eingespeisten 4,4'-Diisocyanatodiphenylmethans enthält. Dieser Sumpfstrom kann in die Trennwandkolonne oder in die Polymer-Monomertrennung zurückgeführt werden. Als Kopfprodukt wird in Abhängigkeit vom Feedstrom ein Gemisch, bestehend aus 2,2'-Diisocyanatodiphenylmethan (max. 80 Gew.-%), 2,4'-Diisocyanatodiphenylmethan (18 bis 80 Gew.-%) und 4,4'-Diisocyanatodiphenylmethan (max. 2 Gew.-%) sowie als Seitenstrom ein Gemisch aus 2,4'-Diisocyanatodiphenylmethan und 4,4'-Diisocyanatodiphenylmethan mit einem Gewichtsverhältnis von 99:1 bis 95:5 erhalten. Erfindungsgemäß bedeutsam ist, dass im Seitenstrom der Gehalt an 2,2'-MDI sehr klein ist, d.h. es werden Konzentrationen von maximal 0,2 Gew.-%, bevorzugt maximal 0,1 Gew.-%, erreicht.

In einer besonders bevorzugten Ausführungsform des erfindungsgemäßen Verfahrens wird im Seitenstrom ein Gemisch aus 95 bis 98 Gew.-% 2,4'-MDI und maximal 5 Gew.-% 4,4'-MDI abgezogen. Durch geeignete Flüssigkeitsaufteilung zwischen Vorfraktionierzone und Hauptfraktionierzone kann die Konzentration von 2,4'-MDI im Seitenstrom in Grenzen von 95 bis 99 Gew.-% variiert werden. Das Rücklaufverhältnis im Kopf wird insbesondere im Bereich von 7,5 bis 250 eingestellt, liegt jedoch besonders bevorzugt im Bereich von 30 bis 90, wobei der Kopfstrom 2 bis 12 Gew.-% bezogen auf den Feedstrom beträgt. Der Sumpfstrom beträgt 20 bis 85 Gew.-%, vorzugsweise 40 bis 60 Gew.-%, des Feedstromes.

Alternativ kann das erfindungsgemäße Verfahren auch mit einer weiteren Seitenstromentnahme unterhalb der ersten Seitenstromentnahme ausgeführt werden. Die zweite Seitenstromentnahme liegt dabei auch in der Hauptfraktionierzone. Hier kann ein Gemisch aus 4,4'-MDI und 2,4'-MDI mit 18 bis 82 Gew.-% 2,4'-MDI entnommen werden. Besonders bevorzugt wird ein kommerziell verwertbares Gemisch aus 50 bis 60 Gew.-% 2,4'-MDI mit maximal 0,2 Gew.-% 2,2'-Diisocyanatodiphenylmethan entnommen.

Nach dem erfindungsgemäßen Verfahren wird das Hauptprodukt, nämlich weitgehend reines 2,4'-MDI, als Seitenstrom der Trennwandkolonne in der Hauptfraktionierzone entnommen. Die Menge des Seitenstromes beträgt bevorzugt mindestens 3 Gew.-% des Feedstroms. Besonders bevorzugt beträgt der Anteil des Seitenstromes 38 bis 50 Gew.-%, z.B. 44%, des Feedstroms. Das Hauptprodukt besteht zu 95 bis 99 Gew.-% aus 2,4'-MDI und maximal 5 Gew.-%, insbesondere 1 bis 5 Gew.-%, 4,4'-MDI, wobei der Anteil an 2,2'-MDI maximal 0,2 Gew.-% beträgt. Besonders bevorzugtes Produkt im Seitenstrom ist ein Gemisch aus 95 bis 98 Gew.-% 2,4'-MDI und 2,5 bis 5 Gew.-% 4,4'-MDI, wobei der Anteil an 2,2'-MDI maximal 0,2 Gew.-% beträgt. Das Sumpfprodukt besteht bevorzugt aus 2 bis 35 Gew.-% 2,4'-MDI und 65 bis 98 Gew.-% 4,4'-MDI, besonders bevorzugt aus 8 bis 12 Gew.-% 2,4'-MDI und 88 bis 92 Gew.-% 4,4'-MDI. In diesem Fall kann das Sumpfprodukt in die vorgeschaltete Destillation (destillative Vortrennung) zurückgeführt werden. Das Kopfprodukt besteht vorzugsweise aus 20 bis 67 Gew.-% 2,2'-MDI, 33 bis 80 Gew.-% 2,4'-MDI, wobei der Anteil an 4,4'-MDI maximal 2 Gew.-% beträgt.

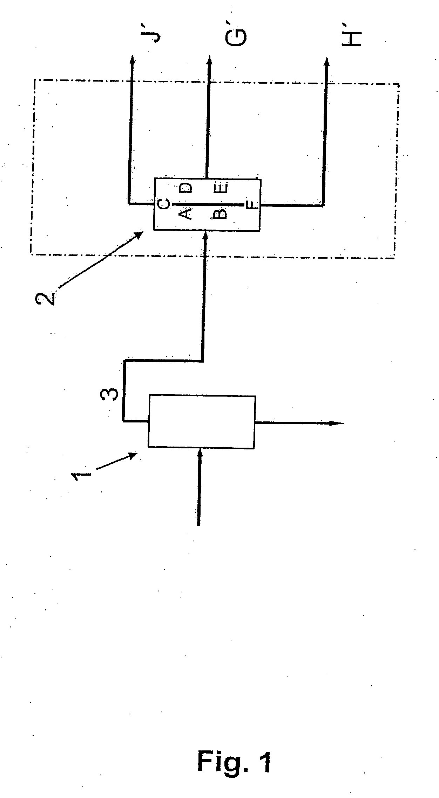

In einer ersten Ausführungsform gemäß Figur 1 erfolgt die Destillation des MDI-Isomerengemisches einstufig mittels einer Trennwandkolonne. Dabei wird ein MDI-Rohgemisch aus der Polymer-Monomerabtrennung (Ausgangsgemisch) der Trennwandkolonne im mittleren Bereich der Trennwand zugeführt. Über die Zone A werden insbesondere chlorhaltige Leichtsieder, Lösungsmittel und der größte Anteil an 2,2'-MDI in die Zone C übergeführt. Des Weiteren werden Anteile des 2,4'-Isomeren in diese Zone transportiert und je nach eingestelltem Rücklaufverhältnis entweder über Kopf abgezogen oder in die Zone D gedrückt. In Zone D treffen diese Anteile auf einen Strom, der die Trennwand am unteren Ende passiert und über die Zonen B und E eine Vortrennung in 2,4'-MDI und 4,4'-MDI erfährt.

Je nach Abnahmestelle in der Hauptfraktionierzone, d.h. im Bereich D, E, können sowohl unterschiedliche Isomerengemische, bestehend aus 2,2'-, 2,4'- und 4,4'-MDI, als auch reine Isomere entnommen werden. Unter reinen Isomeren sind hier Isomere zu verstehen, die kommerziell verwertbar sind und keiner weiteren Trennung mehr bedürfen.

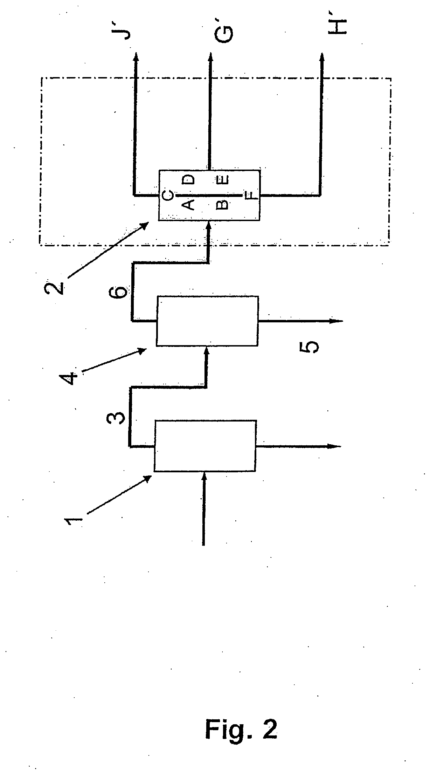

In einer zweiten Ausführungsform gemäß Figur 2b wird der Rohdestillatstrom aus der Polymer-Monomertrennung (Ausgangsgemisch) zunächst in einer ersten Kolonne in einen Kopfstrom, bestehend aus Leichtsieder, 2,2'-MDI, 2,4'-MDI und 4,4'-MDI, sowie einen Sumpfstrom, bestehend aus Resten an 2,4'-MDI und dem Hauptanteil an 4,4'-MDI aufgetrennt.

Der Sumpfstrom dieser ersten Destillationsstufe ohne Trennwandkolonne kann in an sich bekannter Art und Weise in einer nachgeschalteten Kolonne in einen Kopfstrom aus reinem 4,4'-MDI und einen Sumpfstrom aufgetrennt werden, wobei letzterer z.B. wieder in die Polymer-Monomer-Destillation zurückgeführt werden kann.

Der Kopfstrom der ersten Destillationsstufe dient der Gewinnung von an 2,2'-MDI armem 2,4'-MDI und einem an 2,2'-MDI armen 2,4'-/4,4'-MDI-Gemisch mittels Trennwandkolonne. Zu diesem Zweck wird dieser Kopfstrom in die Vorfraktionierzone A, B einer Trennwandkolonne eingespeist. Am Kopf der Trennwandkolonne wird ein Leichsiederstrom entnommen, der den größten Teil des mit dem Feed eingespeisten 2,2'-MDI enthält, während aus der Hauptfraktionierzone D, E ein 2,2'-MDI-armes 2,4'-MDI (max. 0,2% 2,2'-MDI) mit geringem Gehalt an 4,4'-MDI (max. 5%) entnommen werden kann. Über den Sumpf der Trennwandkolonne fällt ein Strom an, der ca. 90 % 4,4'-MDI und ca. 10 % 2,4'-MDI enthält und beispielsweise zur Reingewinnung von 4,4'- MDI einer Kristallisation oder einer weiteren destillativen Trennung zugeführt werden kann.

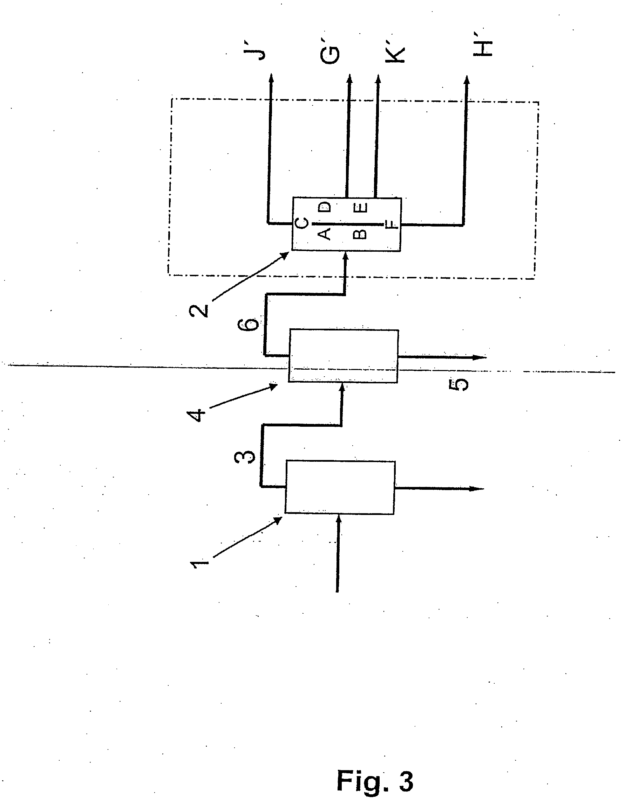

In einer dritten Ausführungsform gemäß Figur 3 wird das MDI-Isomerengemisch ebenfalls zweistufig mit einer Trennwandkolonne in der zweiten Stufe destilliert. Analog zu der zuvor beschriebenen zweistufigen Ausführungsform gemäß Figur 2b wird der Rohdestillatstrom aus der Polymer-Monomertrennung (Ausgangsgemisch) zunächst in einer ersten Kolonne in einen Kopfstrom, bestehend aus Leichtsieder, 2,2'-MDI, 2,4'-MDI und 4,4'-MDI, sowie einen Sumpfstrom, bestehend aus Resten an 2,4'-MDI und dem Hauptanteil an 4,4'-MDI, aufgetrennt.

Der Sumpfstrom der ersten Destillationsstufe kann wieder in an sich bekannter Art und Weise in einer nachgeschalteten Kolonne in einen Kopfstrom aus reinem 4,4'-MDI und einen Sumpfstrom aufgetrennt werden, wobei letzterer beispielsweise in die Polymer-Monomer-Destillation zurückgeführt werden kann.

Der Kopfstrom der ersten Destillationsstufe dient analog zu der oben beschriebenen zweistufigen Destillation gemäß Figur 2 der Gewinnung von an 2,2'-MDI armem 2,4'-MDI und einem an 2,2'-MDI armen 2,4'-/4,4'-MDI-Gemisch in einer Trennwandkolonne. Zu diesem Zweck wird der Kopfstrom der ersten Destillationsstufe in die Vorfraktionierzone A, B einer Trennwandkolonne eingespeist. Am Kopf der Trennwandkolonne wird ein Leichtsiederstrom entnommen, der den größten Teil des mit dem Feed eingespeisten 2,2'-MDI enthält, während aus der Hauptfraktionierzone D, E ein erster Seitenstrom, bestehend aus 2,2'-MDI-armem 2,4'-MDI (maximal 0,2% 2,2'-MDI) mit geringem Gehalt an 4,4'-MDI (maximal 5%), entnommen wird. Anders als in der zweistufigen Destillation gemäß der zweiten Ausführungsform (Fig. 2) wird gemäß der dritten Ausführungsform (Fig. 3) zusätzlich in der Hauptfraktionierzone D, E unterhalb der Entnahmestelle des 2,2'-MDI-armen 2,4'-MDI ein weiterer 2,2'-MDI-armer Seitenstrom, besonders bevorzugt bestehend aus 50 bis 60% 2,4'-MDI, entnommen.

Über den Sumpf der Trennwandkolonne fällt ein Strom an, der ca. 90 % 4,4'-MDI und ca. 10% 2,4'-MDI enthält und beispielsweise zur Reingewinnung von 4,4'- MDI einer Kristallisation oder einer weiteren destillativer Trennung zugeführt werden kann.

### Beispiele

### Beispiel 1

Es wird gemäß Figur 1 eine einstufige Destillation mit einer Trennwandkolonne durchgeführt. Als Stoffaustauschelemente werden in der Trennwandkolonne Gewebepackungen mit 500 m²/m³ spezifischer Oberfläche eingesetzt. 40 Gew.-% der Flüssigkeit wird auf die Vorfraktionierzone und 60 Gew.-% auf die Hauptfraktionierzone geleitet. Die Rektifizierzone und die Abtriebszone besitzen jeweils 8 Trennstufen, die Vorfraktionierungszone besitzt 12 Trennstufen oben und 14 Trennstufen unten, die Hauptfraktionierungszone besitzt 16 Trennstufen oben und 14 Trennstufen unten, wobei mit den Trennstufen oben und unten - hier und im folgenden - die Trennstufen oberhalb und unterhalb der Zuführung des Feedstromes in die Vorfraktionierungszone bzw. der Seitenstromentnahme aus der Hauptfraktionierungszone gemeint ist. Der Kopfdruck beträgt 6 mbar. Der Rücklauf an der Destillatentnahme beträgt 56:1, der Rücklauf an der Seitenstromentnahme beträgt 3,7:1.

12,5 kg/h eines Isomerengemisches, bestehend aus 3,0 Gew.-% 2,2'-MDI, 50,0 Gew.-% 2,4'-MDI und 47,0 Gew.-% 4,4'-MDI, werden der Trennwandkolonne in der Vorfraktionierzone auf der 13. Stufe von oben zugeführt. Der Trennwandkolonne werden drei Produktströme entnommen: 0,6 kg/h Kopfstrom, bestehend aus 56,4 Gew.-% 2,2'-MDI, 43,5 Gew.-% 2,4'-MDI und 0,1 Gew.-% 4,4'-MDI; 5,5 kg/h Seitenstrom, bestehend aus 0,1 Gew.-% 2,2'-MDI, 97,5 Gew.-% 2,4'-MDI und 2,4 Gew.-% 4,4'-MDI; 6,4 kg/h Sumpfstrom mit einer Isomerenreinheit an 4,4'-MDI von 90 % und 10 % 2,4'-MDI. Der Seitenstrom wird nach der 16. Trennstufe der Hauptfraktionierungszone entnommen.

### Beispiel 2

Es wird gemäß Figur 1 eine einstufige Destillation mit einer Trennwandkolonne durchgeführt. Als Stoffaustauschelemente werden in der Trennwandkolonne Gewebepackungen mit 500 m²/m³ spezifischer Oberfläche eingesetzt. 50 Gew.-% der Flüssigkeit wird auf die Vorfraktionierzone und 50 Gew.-% auf die Hauptfraktionierzone geleitet. Die Rektifizierzone und die Abtriebszone besitzen jeweils 8 Trennstufen, die Vorfraktionierungszone besitzt 12 Trennstufen oben und 14 Trennstufen unten, die Hauptfraktionierungszone besitzt 16 Trennstufen oben, 12 Trennstufen in der Mitte und 2 Trennstufen unten. Der Kopfdruck beträgt 6 mbar. Der Rücklauf an der Destillatentnahme beträgt 72:1, der Rücklauf an der oberen Seitenstromentnahme beträgt 8,2:1, der Rücklauf an der unteren Seitenstromentnahme beträgt 3,9:1. Der obere Seitenstrom wird nach der 16. Trennstufe der Hauptfraktionierzone entnommen. Der untere Seitenstrom wird nach der 28. Trennstufe der Hauptfraktionierzone entnommen. Der Kopfdruck beträgt 6 mbar. Der Rücklauf an der Destillatentnahme beträgt 75:1, der Rücklauf an der oberen Seitenstromentnahme beträgt 9,3:1, an der unteren Seitenstromentnahme 4,0:1.

11 kg/h eines Isomerengemisches, bestehend aus 3,0 Gew.-% 2,2'-MDI, 50,0 Gew.-% 2,4'-MDI und 47,0 Gew.-% 4,4'-MDI werden der Trennwandkolonne auf der 13. Stufe von oben in der Vorfraktionierzone im Bereich der Trennwand zugeführt. Der Trennwandkolonne werden vier Produktströme entnommen: 0,6 kg/h Kopfstrom, bestehend aus 53,3 Gew.-% 2,2'-MDI, 46,6 Gew.-% 2,4'-MDI mit maximal 0,1 Gew.-% 4,4'-MDI; 2,6 kg/h aus dem oberen Seitenstrom, bestehend aus 0,1 Gew.-% 2,2'-MDI, 97,5 Gew.-% 2,4'-MDI, 2,4 Gew.-% 4,4'-MDI; 4,5 kg/h eines unteren Seitenstroms, bestehend aus 0,07 Gew.-% 2,2'-MDI, 55 Gew.-% 2,4'-MDI, 44,93 Gew.-% 4,4'-MDI und ein Sumpfstrom von 3,3 kg/h mit einer Isomerenreinheit von 92,8 % 4,4'-MDI und 7,2 % 2,4'-MDI.

## Patentansprüche

1. Verfahren zur Herstellung von an 2,2'-MDI armem 2,4'-MDI durch Destillation entweder eines Ausgangsgemisches isomerer Diisocyanatodiphenylmethane, wenigstens bestehend aus 2,2'-Diisocyanatodiphenylmethan, 2,4'-Diisocyanatodiphenylmethan und 4,4'-Diisocyanatodiphenylmethan, oder eines in einer destillativen Vortrennung des Ausgangsgemisches erhaltenen Gemisches, welches zu 0 bis 15 Gew.-% aus 2,2'-Diisocyanatodiphenylmethan, zu 12 bis 60 Gew.-% aus 2,4'- Diisocyanatodiphenylmethan und zu 25 bis 88 Gew.-% aus 4,4'-Diisocyanatodiphenylmethan besteht, wobei die Destillation mindestens einstufig ausgeführt ist und in mindestens einer Destillationsstufe eine Trennwandkolonne eingesetzt wird, wobei das Ausgangsgemisch oder das nach destillativer Vortrennung vorliegende Diisocyanatodiphenylmethan-Isomerengemisch als Feedstrom vollständig auf die Vorfraktionierzone der Trennwandkolonne geführt wird, **dadurch gekennzeichnet, dass** die Flüssigkeitsaufteilung zwischen Vorfraktionierzone und Hauptfraktionierzone der Trennwandkolonne so vorgenommen wird, dass im Seitenstrom ein Gemisch erhalten wird, welches 95 bis 99 Gew.-% 2,4'-Diisocyanatodiphenylmethan und maximal 0,2 Gew.-% 2,2'-Diisocyanatodiphenylmethan enthält, wobei sich die Gewichtsprozentangaben auf die Zusammensetzung der Isomere beziehen.

2. Verfahren nach Anspruch 1, **dadurch gekennzeichnet, dass** zusätzlich ein Gemisch aus 4,4'-MDI und 2,4'-MDI mit 18 bis 82 Gew.-% 2,4'-MDI erhalten wird.

3. Verfahren nach Anspruch 2, **dadurch gekennzeichnet, dass** ein Gemisch aus 4,4'-MDI und 2,4'-MDI mit 50 bis 60 Gew.-%, 2,4'-MDI und maximal 0,2 Gew.-% 2,2'-MDI erhalten wird.

4. Verfahren nach einem der Ansprüche 1-3, **dadurch gekennzeichnet, dass** die Destillation zweistufig mit einer Trennwandkolonne in der zweiten Stufe ausgeführt wird, wobei der Kopfstrom der ersten Kolonne der Trennwandkolonne als Feedstrom zugeführt wird.

## Claims

1. Process for producing 2,4'-MDI poor in 2,2'-MDI by distillation either of a starting mixture of isomeric diisocyanatodiphenylmethanes at least consisting of 2,2'-diisocyanatodiphenylmethane, 2,4'-diisocyanatodiphenylmethane and 4,4'-diisocyanatodiphenylmethane or of a mixture obtained in a distillative preseparation of the starting mixture and consisting of 0% to 15% by weight of 2,2'-diisocyanatodiphenylmethane, of 12% to 60% by weight of 2,4'-diisocyanatodiphenylmethane and of 25% to 88% by weight of 4,4'-diisocyanatodiphenylmethane, wherein the distillation is configured as an at least single-stage distillation and a dividing wall column is employed in at least one distillation stage, wherein the starting mixture or the diisocyanatodiphenylmethane isomer mixture present after the distillative preseparation is entirely passed onto the prefractionating zone of the dividing wall column as a feed stream, **characterized in that** the liquid division between the prefractionating zone and the main fractionating zone of the dividing wall column is undertaken such that a mixture containing 95% to 99% by weight of 2,4'-diisocyanatodiphenylmethane and not more than 0.2% by weight of 2,2'-diisocyanatodiphenylmethane is obtained in the sidestream, wherein the reported percentages by weight relate to the composition of the isomers.

2. Process according to Claim 1, **characterized in that** in addition a mixture of 4,4'-MDI and 2,4'-MDI comprising 18% to 82% by weight of 2,4'-MDI is obtained.

3. Process according to Claim 2, **characterized in that** a mixture of 4,4'-MDI and 2,4'-MDI comprising 50% to 60% by weight of 2,4'-MDI and not more than 0.2% by weight of 2,2'-MDI is obtained.

4. Process according to any of Claims 1-3, **characterized in that** the distillation is configured as a two-stage distillation with a dividing wall column in the second stage, wherein the tops stream from the first column is sent to the dividing wall column as a feed stream.

## Revendications

1. Procédé de fabrication de 2,4'-MDI pauvre en 2,2'-MDI par distillation soit d'un mélange de départ de diisocyanatodiphénylméthanes isomères, constitué par au moins du 2,2'-diisocyanatodiphénylméthane, du 2,4'-diisocyanatodiphénylméthane et du 4,4'-diisocyanatodiphénylméthane, soit d'un mélange obtenu par une séparation préliminaire par distillation du mélange de départ, qui est constitué par 0 à 15 % en poids de 2,2'-diisocyanatodiphénylméthane, 12 à 60 % en poids de 2,4'-diisocyanatodiphénylméthane et 25 à 88 % en poids de 4,4'-diisocyanatodiphénylméthane, la distillation étant configurée au moins à une étape et une colonne à paroi de séparation étant utilisée dans au moins une étape de distillation, le mélange de départ ou le mélange d'isomères de diisocyanatodiphénylméthane présent après la séparation préliminaire par distillation étant introduit en totalité en tant que courant d'alimentation dans la zone de fractionnement préliminaire de la colonne à paroi de séparation, **caractérisé en ce que** la répartition du liquide entre la zone de fractionnement préliminaire et la zone de fractionnement principale de la colonne à paroi de séparation est réalisée de telle sorte qu'un mélange qui contient 95 à 99 % en poids de 2,4'-diisocyanatodiphénylméthane et au plus 0,2 % en poids de 2,2'-diisocyanatodiphénylméthane soit obtenu dans le courant latéral, les données de pourcentages en poids se rapportant à la composition des isomères.

2. Procédé selon la revendication 1, **caractérisé en ce qu'**un mélange de 4,4'-MDI et de 2,4'-MDI contenant 18 à 82 % en poids de 2,4'-MDI est en outre obtenu.

3. Procédé selon la revendication 2, **caractérisé en ce qu'**un mélange de 4,4'-MDI et de 2,4'-MDI contenant 50 à 60 % en poids de 2,4'-MDI et au plus 0,2 % en poids de 2,2'-MDI est obtenu.

4. Procédé selon l'une quelconque des revendications 1 à 3, **caractérisé en ce que** la distillation est configurée à deux étapes avec une colonne à paroi de séparation dans la deuxième étape, le courant de tête de la première colonne étant introduit dans la colonne à paroi de séparation en tant que courant d'alimentation.
